# FASCICULE DE BREVET EUROPEEN

(11) **EP 2 879 624 B1**
(45) Date de publication et mention de la délivrance du brevet: **21.09.2016**
(21) Numéro de dépôt: 13758904.0
(22) Date de dépôt: 31.07.2013
(51) Int. Cl.: A61F 2/78, A61F 2/50, B29K 21/00

(54) **PROCÉDÉ DE FABRICATION D'UN MANCHON PROTHÉTIQUE SUR MESURE**
VERFAHREN ZUR HERSTELLUNG EINES PROTHESENSCHAFTES ZUM MESSEN
METHOD FOR PRODUCING A PROSTHETIC SLEEVE TO MEASURE

(30) Priorité: 02.08.2012 FR 1202169; 16.08.2012 FR 1202244
(43) Date de publication de la demande: 10.06.2015
(73) Titulaire: Chabloz Composants, 38170 Seyssinet-Pariset (FR)
(72) Inventeur: CHABLOZ, Pierre, 38450 Saint Georges de Commiers (FR)
(74) Mandataire: Talbot, Alexandre
(86) Numéro de dépôt international: PCT/FR2013/000208
(87) Numéro de publication internationale: WO 2014/020245

(56) Documents cités:
- FR-A- 1 532 625
- US-A- 5 258 037
- US-A- 5 824 111
- US-A1- 2008 188 948
- US-B2- 6 918 936

## Description

### Domaine technique de l'invention

L'invention a pour objet un procédé de fabrication d'un manchon prothétique sur mesure pour le membre résiduel d'un amputé à partir d'une préforme thermoformable.

### État de la technique

Pour les amputés ayant perdus une extrémité d'un membre comme un bras ou une jambe, communément appelé membre résiduel, l'usage d'un manchon entre la prothèse et le membre résiduel est couramment employé pour diminuer les frottements et favoriser le contact entre la peau et la prothèse.

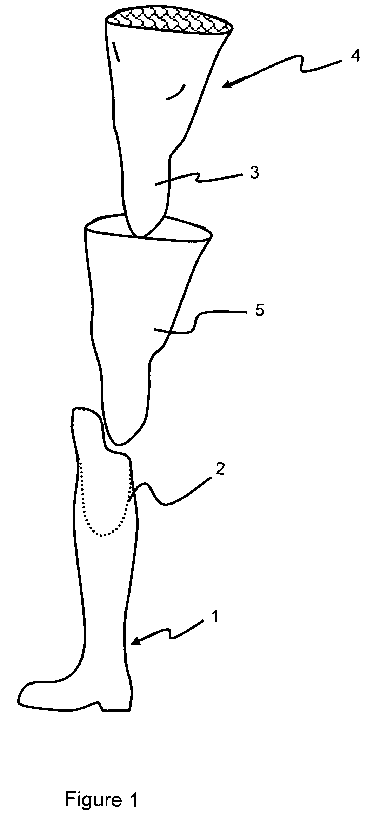

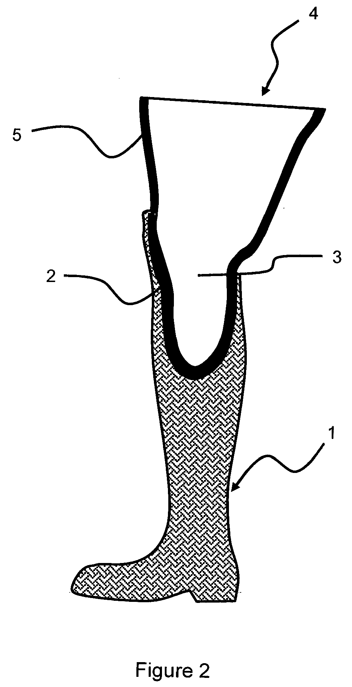

Comme représenté aux figures 1 et 2, une prothèse 1 comporte une emboîture 2 qui est destinée à recevoir le moignon 3 d'un membre résiduel 4 et à le solidariser pour permettre son appui sur la prothèse 1. Un manchon 5 est, généralement, disposé entre le moignon 3 et l'emboîture 2. Le manchon 5 facilite la mise en place du moignon 3 dans la prothèse 1 et améliore le confort de l'amputé.

Lors de la marche prothétique, l'emboîture 2 permet la transmission des forces, au cours de la phase d'appui et l'activation de la prothèse 1 par le moignon 3 du membre résiduel 4.

Les emboîtures 2 sont dites "à contact partiel" lorsque l'extrémité du moignon 3 reste libre, "à succion" lorsqu'il y a une chambre à dépression avec valve ou "à contact total" lorsque tout le moignon 3 est en contact étroit avec l'emboîture 2.

Comme représenté à la figure 2, le manchon 5 peut, avantageusement, être adhérent pour épouser parfaitement la forme du moignon 3 et éviter ainsi les frottements entre le moignon 3 et le manchon 5.

Le manchon 5 doit favoriser une répartition uniforme de la pression ou une répartition dégressive de l'extrémité distale vers l'extrémité proximale du moignon 3 exercée par le membre résiduel 4 sur l'emboîture 2 de la prothèse 1 pour éviter les troubles trophiques et autres complications dues à une défaillance de la circulation artérielle dans le membre résiduel 4.

Par ailleurs, le manchon 5 doit présenter une très bonne adhérence pour assurer son maintien sur le moignon 3 lors de la marche prothétique et être dermatologiquement acceptable afin d'éviter les troubles liées à une intolérance cutanée.

Les manchons adhérents les plus couramment utilisés sont en matériau polymère, sous forme d'une mousse expansée comme les mousses de polyuréthane ou en silicone. Généralement, le matériau polymère utilisé est fortement élastique, présentant une dureté inférieure à 10 Shore A.

Les procédés de fabrication actuels des manchons personnalisés en polyuréthane ou en silicone sont réalisés soit par imprégnation d'un tissu par le matériau polymère soit par coulée du matériau polymère entre un moule positif ayant exactement la forme du moignon 3 et une coque rigide formant un contre-moule négatif. La coque rigide sert de moule extérieur et définit l'épaisseur du manchon 5. Le matériau polymère est alors coulé par gravité ou poussé pour lui permettre d'atteindre le fond du moule.

Ces deux techniques présentent un certain nombre de désavantages. Elles sont difficilement reproductibles et donnent un manchon 5 ayant une épaisseur irrégulière et peu contrôlable. De ce fait, le manchon 5 ainsi obtenu présente des propriétés de serrage difficilement contrôlables et peu reproductibles.

Les brevets US 5258037, FR 1532625 et la demande de brevet US 2008/0188948 divulguent également des manchons prosthétiques en polymère destinés à être placés entre le moignon et la prothèse, les manchons étant configurées pour se conformer à la forme du moignon.

Dans le document US-B-6764631, les auteurs proposent un manchon et un procédé de fabrication remédiant à ces inconvénients. Le procédé consiste à fournir un moule positif s'adaptant exactement à la forme du moignon sur lequel est placé une préforme tubulaire thermoformable puis à mouler ladite préforme tubulaire par chauffage. La préforme thermoformable décrite est constituée d'un élastomère de type gel, formulé par mélange d'un copolymère triblocs styrène-éthylène-styrène avec une huile minérale. Le manchon ainsi obtenu présente, néanmoins, le désavantage d'être collant et de créer des adhérences avec les parois de l'emboîture rendant difficile l'introduction du moignon revêtu du manchon dans l'emboîture de la prothèse. Par ailleurs, le manchon à base d'élastomère gel présente une faible dureté et une faible résistance au déchirement. Ainsi, les auteurs proposent de recouvrir le revêtement thermoformable d'un tissu pour augmenter la durée de vie de ces manchons et faciliter la mise en place du moignon dans l'emboîture. Cette solution n'est pas satisfaisante car elle présente le désavantage d'introduire une étape additionnelle et un coût supplémentaire de fabrication du manchon.

Dans les documents EP-B-1023012 et US-B-6918936, les auteurs proposent un manchon prothétique en un matériau élastomère thermoformable et son procédé de fabrication. Le matériau élastomère thermoformable est préférablement un copolymère Styrène-Ethylène-Butylène-Styrène (SEBS) avantageusement caractérisé par une dureté d'environ 5 à 40 Shore A et une élongation à la rupture d'environ 600 à 1200%. Le procédé quant à lui consiste à fournir un moule positif s'adaptant exactement à la forme du moignon sur lequel est placé un revêtement tubulaire en matériau élastomère thermoformable et à mouler ledit revêtement tubulaire par chauffage à une température d'environ 60°C ou plus. Le manchon ainsi obtenu présente néanmoins le désavantage d'être généralement élastique et donc fortement déformable. Le matériau élastomère SEBS spécifiquement exemplifié, DRYFLEX© 500120 commercialisé par la société Nolato Elastoteknik, Torekov (SE), présente notamment une dureté de 12 Shore A. Les auteurs proposent donc d'augmenter la résistance du manchon par l'adjonction d'un matériau de plus forte dureté, augmentant alors d'autant l'épaisseur du manchon. Par ailleurs, le procédé de fabrication implique l'utilisation d'un moule positif s'adaptant exactement à la forme du moignon, l'adhésion du manchon au moignon étant donc uniquement liée aux propriétés élastiques du manchon et au fait qu'il se rétracte parfois légèrement lors de l'étape de thermoformage. Le manchon et le procédé de fabrication décrits dans le document US-B-6918936 ne permettent donc aucunement une maîtrise précise de la répartition du serrage du manchon sur le moignon.

Il existe donc un besoin insatisfait jusqu'ici d'un manchon prothétique permettant notamment une maîtrise extrêmement précise de la répartition du serrage du manchon sur le moignon.

### Objet de l'invention

L'invention a pour objet un procédé de fabrication d'un manchon prothétique sur mesure qui remédie aux inconvénients de l'art antérieur.

Plus particulièrement, l'invention est relative à un procédé de fabrication d'un manchon prothétique sur mesure durable, confortable, garantissant un drainage amélioré et une bonne circulation au niveau des faisceaux vasculaires et nerveux du membre résiduel tout en assurant une très bonne adhérence entre le moignon et le manchon. Le manchon prothétique obtenu selon le procédé de fabrication de la présente invention permet en particulier une maîtrise extrêmement précise de la répartition du serrage du manchon sur le moignon.

On tend à atteindre cet objectif grâce au procédé de fabrication d'un manchon prothétique sur mesure à partir d'une préforme thermoformable défini dans la revendication 1 annexée, comprenant les étapes successives suivantes :
- fournir un moule positif réduit correspondant à la copie de la forme du moignon du membre résiduel réduite de 3 à 5% de l'ensemble des circonférences de la forme du moignon,
- fournir une préforme à base d'un élastomère thermoformable, ladite préforme présentant une extrémité proximale ouverte, une extrémité distale fermée, une épaisseur de paroi uniforme et une dureté supérieure à 40 Shore A, préférentiellement supérieure à 42 Shore A, plus préférentiellement encore supérieure à 45 Shore A,
- placer la préforme sur le moule positif réduit pour former un ensemble préforme/moule,
- mettre en forme la préforme par chauffage de l'ensemble préforme/moule à une température comprise entre 60°C et 150°C, préférentiellement entre 90°C et 120°C.

### Description sommaire des dessins

D'autres avantages et caractéristiques ressortiront plus clairement de la description qui va suivre de modes particuliers de réalisation de l'invention, donnés à titre d'exemples non limitatifs et représentés aux dessins annexés, dans lesquels :
- La figure 1 représente, schématiquement et en perspective, une prothèse avec manchon selon l'art antérieur.
- La figure 2, représente, schématiquement et en coupe, une prothèse avec manchon selon l'art antérieur.
- Les figures 3 à 7 représentent, schématiquement et en coupe, différentes étapes d'un procédé de fabrication selon un mode de réalisation particulier.

### Description de modes particuliers de réalisation

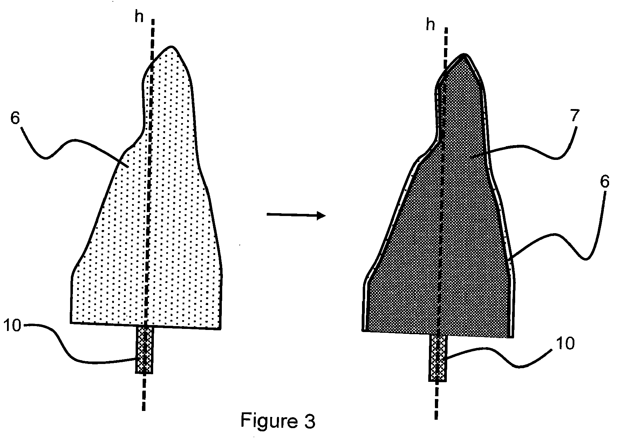

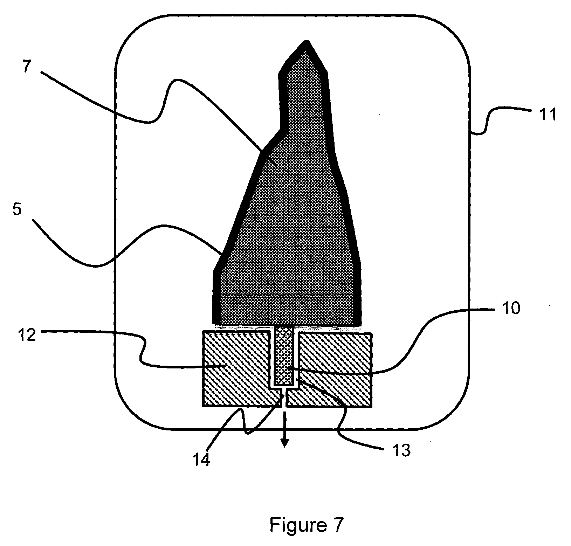

Selon un mode particulier de réalisation représenté aux figures 3 à 7, un procédé de fabrication d'un manchon prothétique 5 sur mesure pour un membre résiduel 4 d'un amputé est mis en oeuvre à partir d'une préforme 8 thermoformable particulière.

De façon surprenante et contre toute attente, il a été découvert que l'utilisation associée d'un moule positif réduit particulier et d'une préforme thermoformable donnée dans un procédé de fabrication d'un manchon prothétique permet d'obtenir un manchon prothétique s'adaptant parfaitement au moignon tout appliquant un serrage précisément déterminé sur ledit moignon.

Le moule positif réduit 7 selon l'invention correspond à la copie parfaite 6 de la forme du moignon 3 du membre résiduel 4 réduite de 3 à 5% de l'ensemble de ses circonférences.

On entend par « réduction de 3 à 5% de l'ensemble des circonférences de la copie 6 de la forme du moignon 3 »,
- soit la réduction de l'ensemble des circonférences de la copie 6 de la forme du moignon 3 d'une valeur donnée, comprise entre 3 et 5% de la valeur desdites circonférences, ces dernières étant perpendiculaires à l'axe h représenté à la figure 3,
- soit la réduction de l'ensemble des circonférences de la copie 6 de la forme du moignon 3 de façon régulière selon un axe proximo-distal par rapport au moignon 3 du membre résiduel 4, la réduction distale étant plus importante que la réduction proximale, la réduction distale étant au maximum de 5% et la réduction proximale étant au maximum de 3%.

Le moule positif réduit 7 peut être réalisé selon tout procédé connu, par exemple, à partir d'une image en 3D de la forme du moignon 3 obtenue par un scanner laser, l'image, copie 6 de la forme du moignon 3, étant ensuite réduite aux dimensions souhaitées et permettant ainsi la réalisation du moule positif réduit 7. La copie 6 de la forme du moignon 3 peut également être réalisée à l'aide du dispositif de mesure décrit dans le document EP-B-1961380.

Le moule positif réduit 7 peut être réalisé en résine ou, de préférence, en plâtre ou en mousse de polyuréthane, à partir d'un moule négatif de la copie 6 de la forme du moignon 3 réduite aux dimensions souhaitées.

Afin de faciliter la réalisation du procédé de fabrication selon l'invention, le moule positif réduit 7 peut posséder un mandrin 10 disposé au centre de la base, non utilisée pour le thermoformage, du moule positif réduit 7. Le mandrin 10 permet le positionnement et le calage ultérieurs du moule positif réduit 7.

La préforme 8 utilisée dans le procédé est constituée à base d'un élastomère thermoformable, c'est-à-dire un élastomère apte à être mis en forme par chauffage. On entend par "à base d'un élastomère thermoformable ", le fait que la préforme 8 est constituée en substance par l'élastomère thermoformable, ce qui n'exclut pas la présence d'éventuels additifs couramment employés dans le domaine.
Les polymères thermoformables sont disponibles dans le commerce sous une large gamme d'élasticité et de dureté. La préforme 8 selon l'invention est constituée à base d'un polymère de dureté supérieure à 40 Shore A, préférentiellement supérieure à 42 Shore A, plus préférentiellement encore supérieure à 45 Shore A. En effet, de façon surprenante, il a été découvert que seuls les élastomères thermoformables sélectionnés de l'invention permettent d'obtenir un manchon prothétique solide, durable, peu élastique, s'adaptant parfaitement au moignon et appliquant un serrage parfaitement déterminé sur ledit moignon. La Demanderesse a constaté, en particulier, qu'une dureté inférieure ou égale à 40 Shore A induisait régulièrement un déchirement du manchon prothétique sur mesure et/ou une trop faible adhérence du manchon au moignon.

L'élastomère thermoformable peut, en particulier, être à base d'un copolymère Styrène-Ethylène-Butylène-Styrène, noté SEBS.

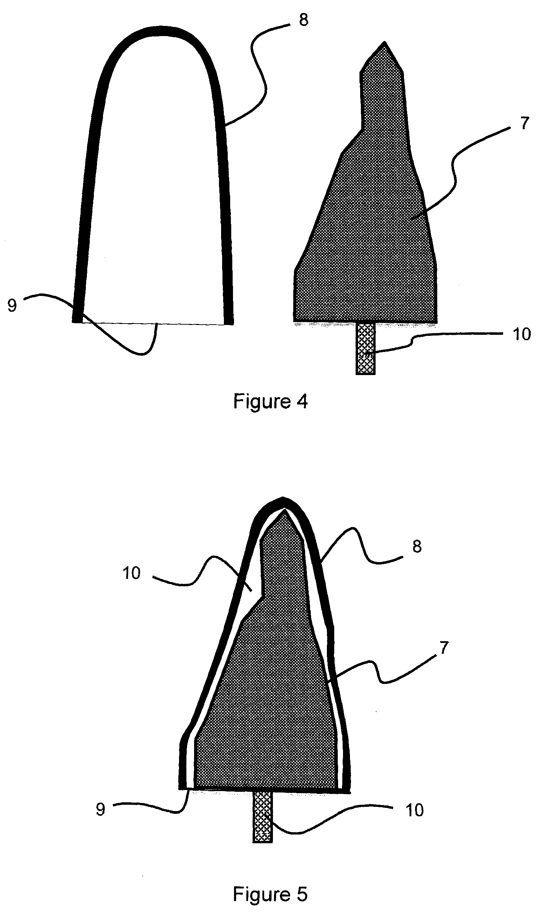

Comme représentée à la figure 4, la préforme 8 présente une extrémité proximale ouverte 9 et une extrémité distale fermée. L'ouverture 9 de l'extrémité proximale est, de préférence, circulaire et présente un diamètre adapté à la dimension du moignon 3 du membre résiduel 4.

La préforme 8 présente, en outre, une épaisseur de paroi uniforme. Préférentiellement, l'épaisseur de paroi varie de 2 à 4 mm.

La préforme 8 peut être obtenue selon tout procédé bien connu de l'homme du métier. Selon un mode préféré de réalisation, la préforme 8 thermoformable est obtenue par injection plastique. Une telle technique d'injection permet d'offrir une large gamme de tailles et de dimensions avec une reproductibilité élevée et une épaisseur de paroi uniforme.

Comme représenté à la figure 5, la préforme 8 est ensuite placée sur le moule positif réduit 7 formant ainsi un ensemble préforme/moule.

La taille de la préforme 8, en particulier, de l'ouverture 9 est choisie de manière à s'adapter aisément à la forme du moule positif réduit 7. La taille de la préforme 8 peut avantageusement être choisie légèrement plus petite que celle du moule positif réduit 7 afin d'exercer une pression suffisante pour maintenir la préforme 8 sur le moule positif réduit 7.

Comme illustré à la figure 5, la paroi de la préforme 8 épouse au moins une partie du moule positif réduit 7, laissant généralement des zones 10 non couvertes du fait de la différence de forme entre la préforme 8 et le moule positif réduit 7.

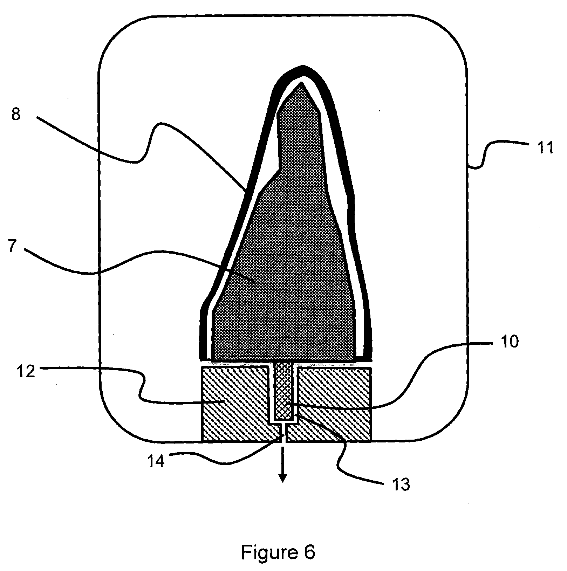

Comme représenté à la figure 6, l'ensemble préforme/moule est ensuite placé dans un four 11, préférentiellement la mise en place est réalisée grâce à un support 12. Le support 12 est classiquement doté d'un logement 13 destiné à recevoir le mandrin 10.

Selon un mode préféré de la présente invention, il existe un dispositif permettant de créer une dépression entre le moule positif réduit 7 et la préforme 8 (non représenté). La mise sous vide au moyen du dispositif de dépression permet une aspiration de l'air, par exemple, à travers un canal 14 formé dans le support 12 du moule positif réduit 7, en communication fluidique avec l'air compris entre le moule positif réduit 7 et la préforme 8 (flèche en bas à la figure 6). La préforme 8 est alors aspirée contre les parois du moule positif réduit 7.
L'éventuelle étape d'aspiration évite la formation de surépaisseurs au niveau des zones 10 non couvertes (figure 5) et améliore la précision de l'ajustement du manchon prothétique 5 et l'uniformité de son épaisseur.

La préforme 8 est ensuite mise en forme par chauffage de l'ensemble préforme/moule à une température comprise entre 60 et 150°C, de préférence entre 90°C et 120°C. L'étape de chauffage est préférentiellement réalisée pendant une durée de 30 à 90 min, préférentiellement de 50 à 75 min. L'élastomère thermoformable formant la préforme 8 se déforme sous l'effet de la chaleur pour épouser alors parfaitement la forme du moule positif réduit 7.

Le manchon prothétique 5 sur mesure est alors obtenu, après retour à température ambiante et, éventuellement, retour à pression atmosphérique entre le moule positif réduit 7 et la préforme 8. Le manchon prothétique 5 sur mesure est démoulé selon toute technique connue, par exemple par découpe de l'extrémité proximale du manchon prothétique 5 et retournement partiel dudit manchon 5.

Selon un mode préférentiellement de la présente invention, le manchon prothétique 5 est revêtu, sur sa face externe, de polyuréthane. Le polyuréthane permet, en effet, de pouvoir enrouler et dérouler facilement le manchon prothétique 5 sur le moignon 3 en garantissant une faible adhérence des différentes portions de la face externe du manchon 5 entre elles. La mise en place et l'enlèvement du manchon prothétique 5 sur le moignon 3 sont ainsi facilités. Préférentiellement, le polyuréthane utilisé sur le manchon prothétique 5 est obtenu par réaction de xylène, d'alcool isobutylique et de diacétone. Un tel polyuréthane permet ainsi un bon compromis entre l'adhérence de différentes portions de la face externe du manchon 5 entre elles et l'adhérence du manchon 5 avec la prothèse 1.

Selon un mode de réalisation préféré, le manchon 5 comprend, entre le polymère thermoformable et le revêtement de polyuréthane, une primaire d'accroche. Préférentiellement, la primaire d'accroche comprend du xylène, de la résine vinyl acétate, de l'acétate de butyle, de l'acétate d'éthyle et un mélange polyoléfine chlorée / chlorobenzène / huile époxydée. En particulier, une primaire d'accroche comprenant les substances ci-avant citées dans les quantités maximales respectives suivantes a présenté d'excellents résultats: max. 30%, max. 5%, max. 50%, max. 20%, max. 20%, (pourcentages en poids).

Selon un mode de réalisation particulier, la préforme 8 est revêtue de polyuréthane et, éventuellement, d'une primaire d'accroche entre le polymère thermoformable et le revêtement de polyuréthane.

Le manchon prothétique 5 peut être solidarisé à la prothèse 1 par l'intermédiaire d'un anneau d'étanchéité (non représenté) solidaire de l'emboîture 2 tel que décrit dans le brevet FR2903294. Alternativement, le manchon prothétique 5 peut classiquement être associé à une valve automatique ou par un dispositif d'accrochage terminal aménagé à l'extrémité du manchon 5 et s'enclipsant dans un mécanisme situé au fond de l'emboîture 2 (non représenté).

Le manchon prothétique 5 ainsi formé présente des qualités d'adhérence et de serrage améliorées ainsi qu'une durée de vie plus élevée que les manchons traditionnels. Le serrage obtenu à partir du manchon prothétique 5 est parfaitement uniforme ou dégressif de l'extrémité distale du moignon 3 vers l'extrémité proximale du moignon 3 garantissant ainsi un excellent drainage du membre résiduel 4. En effet, l'extrémité distale du moignon 3 étant plus serrée que l'extrémité proximale, le drainage lymphatique et sanguin du moignon 3 est ainsi amélioré.

En outre, la bonne adhérence du manchon 5 évite la transpiration et stabilise le moignon 3 en volume, augmentant ainsi, par exemple pour une prothèse 1 de jambe, le périmètre de marche de l'amputé.

Par ailleurs, le procédé de fabrication est facile à mettre en oeuvre et peu coûteux. L'utilisation d'un élastomère thermoformable, préférentiellement à base d'un copolymère SEBS permet d'envisager une production industrielle d'un manchon prothétique 5 sur mesure, avec un rendement élevé et à moindre coût.

## Revendications

1. Procédé de fabrication d'un manchon prothétique sur mesure à partir d'une préforme thermoformable comprenant les étapes successives suivantes:
- fournir un moule positif réduit (7) correspondant à la copie (6) de la forme du moignon (3) du membre résiduel (4) réduite de 3 à 5% de l'ensemble des circonférences de la copie (6) de la forme du moignon (3),
- fournir une préforme (8) à base d'un élastomère thermoformable, ladite préforme (8) présentant une extrémité proximale ouverte (9), une extrémité distale fermée, une épaisseur de paroi uniforme et une dureté supérieure à 40 Shore A,
- placer la préforme (8) sur le moule positif réduit (7) pour former un ensemble préforme/moule,
- mettre en forme la préforme (8) par chauffage de l'ensemble préforme/moule à une température comprise entre 60 et 150°C, préférentiellement entre 90°C et 120°C pour obtenir un manchon prothétique (5).

2. Procédé selon la revendication 1, dans lequel l'élastomère thermoformable présente une dureté supérieure à 42 Shore A, préférentiellement supérieure à 45 Shore A.

3. Procédé selon l'une ou l'autre des revendications 1 et 2, comprenant, après la mise en place du moule positif réduit (7) sur la préforme (8) au moins une étape ultérieure comprenant la création d'une dépression entre le moule positif réduit (7) et la préforme (8).

4. Procédé selon l'une quelconque des revendications 1 à 3, dans lequel l'étape de chauffage est réalisée pendant une durée de 30 à 90 min, préférentiellement de 50 à 75 min.

5. Procédé selon l'une quelconque des revendications 1 à 4, dans lequel la préforme (8) a une épaisseur de paroi uniforme de 2 à 4 mm.

6. Procédé selon l'une quelconque des revendication 1 à 5, dans lequel l'élastomère thermoformable de la préforme (8) est un copolymère Styrène-Ethylène-Butylène-Styrène.

7. Procédé selon l'une quelconque des revendication 1 à 6, dans lequel le manchon prothétique (5) est revêtu de polyuréthane.

## Patentansprüche

1. Verfahren zur Maßanfertigung einer prothetischen Hülse auf der Grundlage eines wärmeformbaren Vorformlings, das folgende aufeinanderfolgende Schritte umfasst:
- Anfertigung einer reduzierten, positiven Form (7), die der Kopie (6) der Form des Stumpfes (3) der Restgliedmaße (4) entspricht, reduziert um 3 bis 5% des Gesamtumfangs der Kopie (6) der Form des Stumpfes (3),
- Anfertigung eins Vorformlings (8) auf Basis eines wärmeformbaren Elastomers, welcher Vorformling (8) ein nahes offenes Ende (9) und ein fernes geschlossenes Ende, eine gleichmäßige Wanddicke und eine Härte von über 40 Shore A aufweist,
- Positionierung des Vorformlings (8) auf der reduzierten positiven Form (7) zur Bildung einer Einheit aus Vorformling und Form,
- Formen des Vorformlings (8) durch Erhitzen der Einheit aus Vorformling und Form bei einer Temperatur von 60 bis 150°C, vorzugsweise 90°C bis 120°C zum Erhalt einer prothetischen Hülse (5).

2. Verfahren nach Anspruch 1, bei dem das wärmeformbare Elastomer eine Härte von über 42 Shore A, vorzugsweise über 45 Shore A, aufweist.

3. Verfahren nach dem einen oder anderen der Ansprüche 1 und 2, das nach dem Positionieren der reduzierten positiven Form (7) auf dem Vorformling (8) mindestens einen weiteren die Herstellung eines Unterdrucks zwischen der reduzierten positiven Form (7) und dem Vorformling (8) umfassenden Schritts umfasst.

4. Verfahren nach einem der Ansprüche 1 bis 3, bei dem der Erhitzungsschritt 30 bis 90, vorzugsweise 50 bis 75 Minuten dauert.

5. Verfahren nach einem der Ansprüche 1 bis 4, bei dem der Vorformling (8) eine Wanddicke von 2 bis 4 mm hat.

6. Verfahren nach einem der Ansprüche 1 bis 5, bei dem das wärmeformbare Elastomer des Vorformlings (8) ein Styren-Ethylen-Butylen-Styren-Kopolymer ist.

7. Verfahren nach einem der Ansprüche 1 bis 6, bei dem die prothetische Hülse (5) mit Polyurethan beschichtet ist.

## Claims

1. A method for manufacturing a made-to-measure prosthetic sleeve from a thermoformable preform comprising the following successive steps:
- providing a reduced positive mould (7) corresponding to the copy (6) of the shape of the stump (3) of the residual limb (4) reduced by 3 to 5% of all of the circumferences of the copy (6) of the shape of the stump (3),
- providing a preform (8) made from a thermoformable elastomer base, said preform (8) presenting an open proximal end (9), a closed distal end, a uniform wall thickness and a hardness of more than 40 Shore A,
- placing the preform (8) on the reduced positive mould (7) to form a preform/ mould assembly,
- shaping the preform (8) by heating the preform/mould assembly to a temperature comprised between 60°C and 150°C, preferably between 90°C and 120°C to obtain a prosthetic sleeve (5).

2. The method according to claim 1, wherein the thermoformable elastomer presents a hardness of more than 42 Shore A, preferably more than 45 Shore A.

3. The method according to either one of claims 1 and 2, wherein, after the reduced positive mould (7) has been placed on the preform (8), it comprises at least one subsequent step comprising creation of a negative pressure between the reduced positive mould (7) and the preform (8).

4. The method according to any one of claims 1 to 3, wherein the heating step is performed for a period of 30 to 90 min, preferably from 50 to 75 min.

5. The method according to any one of claims 1 to 4, wherein the preform (8) has a uniform wall thickness of 2 to 4 mm.

6. The method according to any one of claims 1 to 5, wherein the thermoformable elastomer of the preform (8) is a Styrene-EthyleneButylene-Styrene copolymer.

7. The method according to any one of claims 1 to 6, wherein the prosthetic sleeve (5) is coated with polyurethane.
